(19) Europäisches Patentamt
European Patent Office
Office européen des brevets

(11) **EP 1 965 760 B1**

(12) **EUROPÄISCHE PATENTSCHRIFT**

(45) Veröffentlichungstag und Bekanntmachung des Hinweises auf die Patenterteilung:
**27.02.2013   Patentblatt 2013/09**

(21) Anmeldenummer: **06841326.9**

(22) Anmeldetag: **11.12.2006**

(51) Int Cl.:
*A61K 9/00* (2006.01)       *A61K 9/20* (2006.01)

(86) Internationale Anmeldenummer:
**PCT/EP2006/069514**

(87) Internationale Veröffentlichungsnummer:
**WO 2007/071580 (28.06.2007 Gazette 2007/26)**

(54) **FEINTEILIGES QUERVERNETZTES POLYVINYLPYRROLIDON ALS TABLETTENSPRENGMITTEL**

FINELY DISPERSED CROSS-LINKED POLYVINYLPYRROLIDONE FOR USE AS TABLET DISINTEGRANT

POLYVINYLPYRROLIDONE A RETICULATION TRANSVERSALE ET GRANULOMETRIE FINE SERVANT D'AGENT DE DESINTEGRATION DE COMPRIMES

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **21.12.2005   EP 05112607**
**23.12.2005   EP 05112963**

(43) Veröffentlichungstag der Anmeldung:
**10.09.2008   Patentblatt 2008/37**

(73) Patentinhaber: **BASF SE**
**67056 Ludwigshafen (DE)**

(72) Erfinder:
• KOLTER, Karl
  67117 Limburgerhof (DE)
• FUSSNEGGER, Bernhard
  67489 Kirrweiler (DE)
• KERBER, Michael
  69469 Weinheim (DE)
• ARMBRUSTER, Harald
  67117 Limburgerhof (DE)
• FOLTTMANN, Hubertus
  69121 Heidelberg (DE)
• WIDMAIER, Ralf
  67061 Ludwigshafen (DE)
• PIEROBON, Marianna
  67063 Ludwigshafen (DE)

(56) Entgegenhaltungen:
EP-A- 1 036 839       WO-A-03/032978
WO-A-03/072084       WO-A2-2005/105049

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft die Verwendung von feinteiligem quervernetzten Polyvinylpyrrolidin mit einer mittleren Teilchengröße von 5 bis 60 $\mu$m und einer Hydratationskapazität bestimmt nach der Methode via beschrieben auf Seite 2, zeilen 10-16, größer 7 g/g als Tablettensprengmittel.

Die Verwendung von Sprengmitteln zur Verbesserung des zerfalls und der Auflösungsgeschwindigkeit von Tabletten ist seit langem allgemein bekannt.

Eines der am häufigsten verwendeten Sprengmittel ist dabei quervernetztes Polyvinylpyrrolidon. Solche quervernetzten Polyvinylpyrrolidone sind kommerziell erhältlich, beispielsweise als Kollidon® CL-Typen der Firma BASF Aktiengesellschaft oder als Polyplasdone® XL-Typen der Firma ISP Investments Inc..

Quervernetztes Polyvinylpyrrolidon wird hauptsächlich in relativ grober Form als Tablettensprengmittel eingesetzt (Kollidon CL im Bereich von 120 $\mu$m und Polyplasdone XL im Bereich von 130 $\mu$m), wobei die mittlere Teilchengröße größer 100 $\mu$m beträgt. Die große Teilchengröße führt zwar zu einer guten Sprengmittelwirkung, bringt jedoch zahlreiche Nachteile wie rauere Tablettenoberfläche bei feuchter Lagerung, niedrige Bruchfestigkeit, Abnahme der Bruchfestigkeit bei feuchter Lagerung, inhomogene Tablettenmischung und Entmischungsneigung in der Tablettiermischung mit sich. Hinzu kommt, dass die groben Sprengmittelteilchen ein sandiges und unangenehmes Mundgefühl verursachen.

Nach allgemeiner Auffassung wurde die Sprengmittelwirkung bisher immer mit der Teilchengröße korreliert. Danach sollten kleinere Teilchen immer eine erheblich reduzierte Sprengmittelwirkung ergeben (siehe V. Buehler, "Polyvinylpyrrolidone Excipients for Pharmaceuticals", S. 128 ff., Springer Verlag Berlin Heidelberg, 2005. So sind die Sprengmittelwirkungen von Polyplasdone XL 10 (Teilchengrößen im Bereich von 30 $\mu$m) und von Kollidon CL-M ( Teilchengrößen im Bereich von 5$\mu$m) erheblich niedriger. Zur Erzielung einer bestimmten Sprengmittelwirkung sind daher bei diesen kleinteiligen Produkten viel höhere Konzentrationen erforderlich, was wiederum nachteilig ist, da höhere Kosten entstehen und die Tabletten feuchtempfindlicher werden. Sehr häufig kann selbst durch eine Konzentrationserhöhung kein so schneller Zerfall erreicht werden wie unter Verwendung von grobteiligem quervernetztem Polyvinylpyrrolidon.

WO2005105049, WO03072084, WO03032978 and EP1036839 offenbaren Polyvinylpyrrolidone in schnell zerfallenden Tablettenformulierungen.

Aus der US 6,677,417 ist die Verwendung von quervernetzten Polyvinylpyrrolidonen mit Teilchengrößen von > 400 $\mu$m bis zu 1500 $\mu$m als Tablettensprengmittel bekannt.

Aufgabe der vorliegenden Erfindung war es, Tablettensprengmittel zu finden, die die geschilderten Nachteile vermeiden helfen.

Demgemäß wurde die eingangs definierte Verwendung gefunden. Die erfindungsgemäß verwendeten Sprengmittel aus quervernetztem Polyvinylpyrrolidon besitzen eine mittlere Teilchengröße von 5 - 60$\mu$m, vorzugsweise 10 - 50$\mu$m und besonders bevorzugt 15 - 40$\mu$m. Die Hydratationskapazitäten betragen größer 7,0g/g, bevorzugt größer 7,5g/g und besonders bevorzugt größer 8,0g/g, und können bis zu 12,0g/g betragen.

Die Bestimmung der Hydratationskapazität erfolgt nach folgender Methode:
2g Polymer werden in ein Zentrifugenglas eingewogen und mit 40ml Wasser 15 Minuten ausquellen lassen. Anschließend wird 15 Minuten bei 2000U/Min zentrifugiert, die überstehende Flüssigkeit abgegossen und die Probe erneut ausgewogen.

$$\text{Hydratationskapazität} = \frac{\text{Auswaage-Tara}}{\text{Einwaage}}$$

(siehe auch V. Buehler, "Polyvinylpyrrolidone Excipients for Pharmaceuticals", S. 132 ff., Springer Verlag Berlin Heidelberg, 2005)

Die Herstellung solcher quervernetzten Polyvinylpyrrolidone kann durch ein an sich bekanntes als "Popcornpolymerisation" bezeichnetes Polymerisationsverfahren erfolgen. Die Popcornpolymerisation wird nach bekannten Verfahren durchgeführt, z. B. als Fällungspolymerisation oder durch Polymerisieren in Substanz. Bekannt ist beispielsweise eine Arbeitsweise, bei der man - wie in der EP-B-0 177 812 beschrieben - die Popcornpolymerisation dadurch startet, daß man eine Mischung aus 99,6 bis 98,8 Gew.-% N-Vinylpyrrolidon und 0,4 bis 1,2 Gew.-% einer mindestens zwei ethylenisch ungesättigte Doppelbindungen aufweisenden Verbindung als Vernetzer auf eine Temperatur in dem Bereich von 100 bis 150 °C in Abwesenheit von Sauerstoff und Polymerisationsinitiatoren erhitzt.

Als Vernetzer werden Verbindungen eingesetzt, die mindestens zwei ethylenisch ungesättigte Doppelbindungen im Molekül enthalten. Besonders geeignet sind beispielsweise Alkylenbisacrylamide wie Methylenbisacrylamid und N,N'-Acryloylethylendiamin, N,N'-Divinylethylenharnstoff, N,N'-Divinylpropylenharnstoff, Ethylidenbis-3-(N-vinylpyrrolidon), N,N'-Divinyl-diimidazolyl(2,2')butan und 1,1'-bis-(3,3'-vinylbenzimidazolyl-2-on)1,4-butan. Andere geeignete Vernetzer sind beispielsweise Alkylenglykoldi(meth)acrylate wie Ethylenglykoldiacrylat, Ethylenglykoldimethacrylat, Tetraethylenglykolacrylat, Tetraethylenglykoldimethacrylat, Diethylenglykolacrylat, Diethylenglykolmethacrylat, aromatische Divinylverbindungen wie Divinylbenzol und Divinyltoluol sowie Vinylacrylat, Allylacrylat, Allylmethacrylat, Divinyldioxan, Pentaerythrittriallylether sowie Gemische der Vernetzer. Der bevorzugt eingesetzte Vernetzer ist N,N'-Divinylethylenharnstoff.

Die Vernetzer werden in Mengen von 0,1 bis 10, vorzugsweise I bis 4 Gew.-%, bezogen auf die bei der Polymerisation eingesetzten Monomeren angewendet.

Diese Polymerisation kann durch Anwesenheit geringer Mengen an Natronlauge oder Kalilauge initiiert werden. Innerhalb einer kurzen Zeit bildet sich ein polymerisationsfähiges Popcornpolymerisat, das bei Zugabe von weiterem N-Vinylpyrrolidon und weiterhin Zugabe des Vernetzers die Popcornpolymerisation ohne Induktionsperiode startet und vervollständigt, wobei man erfindungsgemäß zur Regelung der Teilchengröße der entstehenden Popcornpolymerisate einen Inertgasstrom vorzugsweise in die im Polymerisationsgefäß vorgelegten Monomeren bereits vor Beginn der Popcornpolymerisation leitet. Mit dem Einleiten des Inertgasstroms wird jedoch spätestens während der Induktionsperiode der Polymerisation begonnen, wobei der Inertgasstrom während der gesamten Dauer der Popcornpolymerisation durch die Reaktionsmischung geleitet wird.

Um die Popcornpolymerisation ohne Lösemittel, d.h. in Substanz, durchzuführen, wird das vorgelegte Monomer durch Einleiten von Stickstoff inertisiert und anschließend auf eine Temperatur in dem Bereich von 100 bis 200, vorzugsweise 150 bis 180°C erhitzt. Es ist vorteilhaft, auch während der Polymerisation weiter einen schwachen Stickstoffstrom durch die Monomeren zu leiten. Ausschluß von Sauerstoff wird auch dadurch erreicht, daß man den Ansatz bei einem Druck polymerisiert, der unter dem Atmosphärendruck liegt und bei dem die Monomeren sieden. Man kann jedoch die Popcornpolymerisation unter vermindertem Druck und gleichzeitigem Einleiten eines Inertgases durchführen. Je nach Menge des eingesetzten Monomeren und der gewählten Temperatur polymerisiert die Mischung innerhalb von 1 bis 20 Stunden. Das Popcornpolymerisat wird daraus in Ausbeuten von über 90% in Form eines Pulvers erhalten.

Zur Herstellung der Popcornpolymerisate wird jedoch die Fällungspolymerisation in Wasser bevorzugt. Die Konzentration des Monomeren wird dabei zweckmäßigerweise so gewählt, daß das Reaktionsgemisch über die gesamte Reaktionsdauer hinweg gut gerührt werden kann. Bei einer zu hohen Konzentration des Monomeren in Wasser, z. B. bei 95 Gew.-%, werden die Polymerisatkömer klebrig, so daß ein Rühren schwieriger wird als in verdünnterer wässriger Lösung. Um die Reaktion in den üblichen Rührkesseln durchzuführen, wählt man beispielsweise Monomerkonzentrationen, bezogen auf die wässrige Mischung, von etwa 5 bis 30, vorzugsweise 10 bis 20 Gew.-%. Falls kräftigere Rührwerke zur Verfügung stehen, kann die Monomerkonzentration der wässrigen Lösung auch bis auf 50 Gew.-%, gegebenenfalls auch darüber erhöht werden. In einigen Fällen kann es zweckmäßig sein, die Popcornpolymerisation mit einer relativ konzentrierten Lösung zu beginnen und dann im Verlauf der Reaktion Wasser zur Verdünnung zuzusetzen.

Die Popcornpolymerisation wird bevorzugt bei pH-Werten oberhalb von 6 durchgeführt, um eine eventuell mögliche Hydrolyse der Monomeren zu vermeiden. Die Einstellung des pH-Wertes kann durch Zugabe geringer Mengen an Basen wie Natriumhydroxid oder Ammoniak oder der üblichen Puffersalze wie Soda, Natriumhydrogencarbonat oder Natriumphosphat erfolgen. Gegebenenfalls kann der Ausschluß von Sauerstoff dadurch erreicht werden, daß man das zu polymerisierende Gemisch zum Sieden erhitzt und zusätzlich einen Inertgasstrom zur Regelung der Teilchengröße der Popcorn-polymerisate durch das Reaktionsgemisch leitet.

Die Teilchengröße der Popcornpolymerisate wird dabei durch die Menge des Inertgasstromes geregelt, der durch die Polymerisationslösung geleitet wird. Wird dem Reaktionsansatz wenig Inertgas zugeführt, so entstehen in der Regel grobe Popcornpolymerisate, wird die Polymerisationslösung mit viel Inertgas durchströmt, so entstehen feinere PopcornPolymerisate. In Abhängigkeit von verschiedenen Rahmenbedingungen wie z.B. Kesselgröße, Temperatur und Druck, können für den jeweiligen Einzelfall keine exakten Bedingungen für die Einstellung einer bestimmten Teilchengröße für die Popcornpolymmerisate angegeben werden. Die für den Einzelfall richtige Menge an Inertgas, die durch die Reaktionsmischung geleitet werden muß, kann durch wenige einfache Versuche leicht ermittelt werden. Wie oben bereits angegeben, liegen die durch die Reaktionsmischung zu leitenden Mengen an Inertgas in dem Bereich von 0,01 bis 100, vorzugsweise 0,1 bis 30 l Inertgas/ I Reaktionsgemisch/ h.

Als Inertgas können Edelgase wie Helium, Neon oder Argon verwendet werden. Auch Kohlendioxid ist geeignet. Vorzugsweise wird Stickstoff verwendet.

Weiterhin können dem Reaktionsgemisch zur völligen Entfernung von gelöstem Sauerstoff geringe Mengen - z.B. 0,1 bis 1 Gew.-%, bezogen auf die Monomermischung - eines Reduktionsmittels wie Natriumsulfit, Natriumpyrosulfit, Natriumdithionit, Ascorbinsäure oder Mischungen der Reduktionsmittel, zugesetzt werden. Gemäß einer bevorzugten Ausführungsform erfolgt die Popcorn-Polymerisation in Gegenwart von Natriumdithionit als Reduktionsmittel.

Die Polymerisationstemperatur kann in einem weiten Bereich variiert werden, z. B. von etwa 20 bis 200, vorzugsweise 50 bis 150°C.

Bei einer besonders bevorzugten Ausführungsform der Fällungspolymerisation wird ein wasserlösliches Comonomer, ein Teil des Vernetzers, Wasser und gegebenenfalls ein Puffer und ein Reduktionsmittel in einem Inertgasstrom erhitzt, bis sich die ersten Polymerisatteilchen zeigen. Dann wird - wenn gewünscht - eine vorher durch Einblasen von Stickstoff inertisierte Mischung aus einem oder mehreren der oben genannten Comonomeren und dem restlichen Vernetzer und gegebenenfalls Wasser als Verdünnungsmittel innerhalb von 0,2 bis 5 Stunden zugegeben. Diese Arbeitsweise hat den Vorteil, daß die Popcornpolymerisation nur kurze Zeit in Anspruch nimmt.

Die Popcornpolymerisate fallen üblicherweise mit einer Ausbeute von etwa 90 bis > 99% der theoretischen Ausbeute an. Sie können aus der wäßrigen Suspension durch Filtrieren oder Zentrifugieren mit anschließendem Auswaschen mit Wasser und Trocknen in üblichen Trocknern wie Umluft- oder Vakuumtrockenschrank, Schaufeltrockner, Taumeltrockner, Wirbelschichttrockner oder Stromtrockner isoliert werden. Die Popcornpolymerisate sind in Wasser und allen Lösemitteln nicht löslich und quellen darin auch nur geringfügig. Die Quellvolumina in Wasser liegen im Bereich von 8 bis 10 l/kg. Erfindungsgemäß geeignete Popcorn-Polymerisate sind kommerziell als Divergan®-Typen der Fa. BASF Aktiengesellschaft erhältlich.

Das Verfahren wird so geführt, dass der mittlere Teilchendurchmesser der getrockneten Polymerisate liegt im Bereich von 5 μm bis 60 μm liegt und die Hydratationskapazität grösser 7 g/ g beträgt. Die Bestimmung der Teilchengrößen kann durch Lichtstreuungsmessungen mit einem Malvern Mastersizer erfolgen.

Diese Polymeren lassen sich sehr homogen in Tablettiermischungen einmischen und führen so zu erheblich reduzierten Standardabweichungen der Zerfallszeiten von Tabletten. Die Tablettenoberfläche ist wesentlich glatter, selbst bei feuchter Lagerung.

In besonderer Weise sind die erfindungsgemäßen Polymeren für im Mund schnell zerfallende Tabletten sogenannte "fast dispersible" oder "fast meltable" tablets geeignet, weil im Gegensatz zu den groben Sprengmitteln kein sandiges, sondern sogar ein sehr weiches angenehmes Mundgefühl erzeugt wird.

Gleichermaßen vorteilhaft sind sie für Tabletten, die vor der Einnahme in einem Glas Wasser zerfallen gelassen werden. Hier bildet sich kein körniger Bodensatz aus, sondern es entsteht eine feine Suspension.

Hinzu kommt, dass sie mit sehr großen Mengen an Wasser oder organischen Lösungsmitteln versetzt werden können, ohne dass sie feucht werden oder eine Paste beziehungsweise einen Brei ergeben. Diese Eigenschaft ist besonders bei der Feuchtgranulation im Mischer, vor allem wenn auch noch höhere Sprengmittelkonzentrationen und höhere Bindemittelkonzentrationen verwendet werden sollen, vorteilhaft. Überraschenderweise verlieren die erfindungsgemäßen Polymere nicht an Sprengmittelwirkung durch Befeuchtung und Trocknung, d.h. sie können mitgranuliert werden und die Tabletten zerfallen trotzdem schnell. Auch daher sind sie in besonderer Weise für die Feuchtgranulation geeignet.

Üblicherweise werden die erfindungsgemäß verwendeten Sprengmittel in Mengen von 0,5 bis 50 Gew.-%, bevorzugt 1 bis 10 Gew.-%, bezogen auf das Tablettengewicht, eingesetzt.

Grundsätzlich eignen sich die erfindungsgemäß verwendeten Sprengmittel zur Herstellung von Tabletten für alle Klassen von pharmazeutischen Wirkstoffen. Weiterhin eignen sie sich auch zur Herstellung von Nahrungsergänzungsmitteln in Tablettenform.

Bevorzugt eignen sich die erfindungsgemäß verwendeten Sprengmittel zur Herstellung von schnell zerfallenden Tabletten. Schnell zerfallend bedeutet, dass die Tabletten in Wasser bei 20 °C innerhalb von 10 bis 120 Sekundenvollständig zerfallen.

Insbesondere eignen sich die erfindungsgemäß verwendeten Sprengmittel zur Herstellung von im Mund schnell zerfallenden Tabletten.

Eigenschaften der erfindungsgemäß verwendeten Polymere (Polymere 1, 2) und der Vergleichsprodukte:

| | Kollidon CL | Kollidon CL-M | Polyplasdone XL 10 | Polymer.1 | Polymer 2 |
|---|---|---|---|---|---|
| Hydratationskapazität [g/g] | 4,4 | 4,0 | 4,6 | 8,0 | 8,1 |
| Mittlere Teilchen-Größe [μm] | 120 | 5 | 29 | 19 | 21 |

Anwendungsbeispiele

Placebotabletten

Zusammensetzung der Tablette bei einer Sprengmittelkonzentration von 6%:

| | |
|---|---|
| Ludipress LCE | 467,50mg |
| Sprengmittel | 30,00mg |
| Mg-Stearat | 2,50mg |
| Tablettengewicht | 500,00mg |

Alle Bestandteile wurden über ein 0.8mm Sieb gegeben und in einem Turbulamischer (Fa. Bachofen, Schweiz) 10min gemischt. Die Tablettierung erfolgte auf einer Excenter- Presse (Korsch EKO, Fa. Korsch, Berlin) bei 30Hüben/min unter Verwendung eines 12mm- Stempels (facettiert). Die Presskraft betrug 18 kN.

Folgende Tabletteneigenschaften wurden erzielt:

| | Ohne Sprengmittel | Kollidon CL | Kollidon CL-M | Polyplasdone XL | Polyplasdone XL 10 | Polymer [1] |
|---|---|---|---|---|---|---|
| Zerfall (s) | 433 | 70 | 291 | 73 | 127 | 67 |
| Rel. Standardabweichung Zerfall (%) | 15,7 | 14,5 | 13,9 | 14,0 | 6,3 | 6,5 |
| Bruchfestigkeit (N) | 165 | 145 | 194 | 148 | 161 | 178 |
| Friabilität (%) | 0,19 | 0,11 | 0,05 | 0,16 | 0,12 | 0,05 |

Stabilitätsprüfung der Tabletten bei 23°C/65% r.F über 7 Tage:

Tablette mit Kollidon CL:     6,5
Tablette mit Polymer gem. Bsp. 1     4

Beurteilungskriterien für die Tablettenoberfläche:

1     glatt
2     kleine Unebenheiten auf der Tablettenoberfläche
3     kleine Unebenheiten/ rauhe Tablettenoberfläche
4     auffällige Unebenheiten/ beginnende Pickelbildung
5     leichte Pickelbildung
6     mittlere Pickelbildung
7     starke Pickelbildung
8     starke Pickelbildung/ Tablette brüchig und aufgeschwemmt

Schmerztabletten

Zusammensetzung der Tablette bei einer Sprengmittelkonzentration von 2,7 Gew.-%:

| Acetylsalicylsäure-Pulver | 250,00mg |
|---|---|
| Paracetamol krist | 250,00mg |
| Coffein gran. 0,2-0,5 | 50,00mg |
| Kollidon 30 | 27,50mg |
| Sprengmittel | 16,00mg |
| Mg-Stearat | 5,00mg |
| Tablettengewicht | 598,50mg |

Zunächst wurden die drei Wirkstoffe in einem Diosnamischer gemischt und mit einer 20%igen wässrigen Kollidon 30 Lösung befeuchtet. Anschließend wurde die feuchte Masse durch ein Sieb einer Maschenweite 0,8mm gegeben und 24h bei Raumtemperatur in dünner Schicht auf einer Horde getrocknet. Dieses Granulat wurde mit dem Sprengmittel und Magnesiumstearat versetzt, über ein 0.8mm Sieb gegeben und in einem Turbulamischer (Fa. Bachofen, Schweiz) 10min gemischt. Die Tablettierung erfolgte auf einer Excenter- Presse (Korsch EKO, Fa. Korsch, Berlin) bei 30Hüben/min unter Verwendung eines 12mm- Stempels (facettiert). Die Presskraft betrug 18kN.

Folgende Tabletteneigenschaften wurden erzielt:

|  | Ohne Sprengmittel | Kollidon CL | Kollidon CL-M | Polyplasdone XL | Polyplasdone XL 10 | Polymer gem. Bsp. 1 |
|---|---|---|---|---|---|---|
| Zerfall (min:s) | 70:40 | 9:13 | 18:15 | 13:03 | 19:04 | 7:36 |
| Bruchfestigkeit (N) | 165 | 93 | 120 | 97 | 106 | 149 |
| Friabilität (%) | 0,38 | 0,38 | 0,20 | 0,41 | 0,42 | 0,25 |

Stabilitätsprüfung der Tabletten bei 23°C/85% r.F über 7 Tage:

| | |
|---|---|
| Kollidon CL: | 6,5 |
| Crospovidone Nr.1 | 3 |
| Hydratationskapazität 8,0 g/g | |

Vitamin C-Tabletten

Zusammensetzung der Tablette bei einer Sprengmittelkonzentration von 3,2 Gew.-%:

| | |
|---|---|
| Vitamin C 90 (Roche) | 480,00mg |
| Avicel PH 102 | 96,06mg |
| Sprengmittel | 19,20mg |
| Mg-Stearat | 4,32mg |
| Tablettengewicht | 599,58mg |

Alle Bestandteile wurden über ein 1,0mm Sieb gegeben und in einem Turbulamischer (Fa. Bachofen, Schweiz) 10min gemischt. Die Tablettierung erfolgte auf einer Rundläufer- Presse (Korsch PH 106, Fa. Korsch, Berlin) bei 40U/min unter Verwendung eines 12mm- Stempels (facettiert). Die Presskraft betrug 18kN.

Folgende Tabletteneigenschaften wurden erzielt:

|  | Ohne Sprengmittel | Kollidon CL | Kollidon CL-M | Polyplasdone XL | Polyplasdone XL 10 | Polymer gem. Bsp. 1 |
|---|---|---|---|---|---|---|
| Zerfall (min:s) | 11:29 | 3:57 | 6:12 | 4:53 | 6:00 | 4:54 |
| Bruchfestigkeit (N) | 141 | 114 | 207 | 131 | 144 | 200 |
| Friabilität (%) | 0,21 | 0,21 | 0,12 | 0,14 | 0,15 | 0,11 |

Stabilitätsprüfung der Tabletten bei 23°C/65% r.F über 7 Tage:

| | |
|---|---|
| Kollidon CL: | 6 |
| Crospovidone Nr.1 | 2,5 |
| Hydratationskapazität 8,0 g/g | |

Oral zerfallende Loratadin Tablette

Zusammensetzung der Tablette bei einer Sprengmittelkonzentration von 8,0%:

| | |
|---|---|
| Loratadin | 10,0mg |
| Mannitol | 100,0mg |
| Erythritol | 73,0mg |
| Sprengmittel | 16,0mg |
| Mg-Stearat | 1,0mg |
| Tablettengewicht | 200,0mg |

Alle Bestandteile wurden über ein 0,8mm Sieb gegeben und in einem Turbulamischer (Fa. Bachofen, Schweiz) 10min gemischt. Die Tablettierung erfolgte auf einer Rundläufer- Presse (Korsch PH 106, Fa. Korsch, Berlin) bei 40U/min unter Verwendung eines 8mm- Stempels (facettiert). Die Presskraft betrug 15kN.
Folgende Tabletteneigenschaften wurden erzielt:

| | |
|---|---|
| Zerfall im Mund: | 40s |
| Mundgefühl: | sehr angenehm, leicht kühlend, nicht körnig |
| Bruchfestigkeit: | 50N |

Schnell zerfallende Acetylsalicylsäure - Tablette

Zusammensetzung der Tablette bei einer Sprengmittelkonzentration von 8,0 Gew.-%:

| | |
|---|---|
| Acetylsalicylsäure | 100,0mg |
| Mannitol | 60,0mg |
| Avicel PH 101 | 53,5,0mg |
| Sprengmittel | 35,0mg |
| Mg-Stearat | 1,5mg |
| Tablettengewicht | 250,0mg |

Alle Bestandteile wurden über ein 0,8mm Sieb gegeben und in einem Pflugscharmischer (Fa. Lödige) 5min gemischt. Die Tablettierung erfolgte auf einer Rundläufer-Presse (Korsch PH 106, Fa. Korsch, Berlin) bei 30U/min unter Verwendung eines 10mm- Stempels (facettiert). Die Presskraft betrug 16kN.
Folgende Tabletteneigenschaften wurden erzielt:

| | |
|---|---|
| Bruchfestigkeit: | 70N |
| Zerfall im Glas Wasser (200ml): | 60s, Aussehen: feine Suspension |

**Patentansprüche**

1.   Verwendung von quervernetztem Polyvinylpyrrolidon mit einer mittleren Teilchengröße von 5 - 60$\mu$m und einer Hydratationskapazität bestimmt nach der Methode wie beschrieben auf Seite 2, Zeilen 10 bis 16 von größer 7 g/g als Tablettensprengmittel.

2.   Verwendung nach Anspruch 1, wobei die Teilchengrößen 10 bis 50$\mu$m beträgt.

3.   Verwendung nach Anspruch 1, wobei die Teilchengröße 15 bis 40$\mu$m beträgt.

4.   Verwendung nach einem der Ansprüche 1 bis 3, wobei die Hydratationskapazität größer als 7,5 g/g ist.

5.   Verwendung nach einem der Ansprüche 1 bis 3, wobei Hydratationskapazität größer als 8,0 g/g ist.

**6.** Tabletten, enthaltend ein quervernetztes Polyvinylpyrrolidon nach einem der Ansprüche 1 bis 5 in Konzentrationen von 0,5 bis 50 Gew.-%, bezogen auf das Gesamtgewicht der Tablette.

**7.** Tabletten nach Anspruch 6, enthaltend ein quervernetztes Polyvinylpyrrolidon in Konzentrationen von 1,0 bis 10 Gew.-%.

**8.** Im Mund schnell zerfallende Tabletten enthaltend ein quervernetztes Polyvinylpyrrolidon nach einem der Ansprüche 1 bis 5 in Konzentrationen von 1,0 bis 10 Gew.-%.

**9.** Im Mund schnell zerfallende Tabletten nach Anspruch 8, enthaltend 20 bis 95 Gew.-% eines Zuckeralkohols.

**Claims**

**1.** The use of crosslinked polyvinylpyrrolidone with an average particle size of 5-60 $\mu$m and a hydration capacity determined by the method as described as page 2, lines 10 to 16, of greater than 7 g/g as tablet disintegrant.

**2.** The use according to claim 1, where the particle size is from 10 to 50 $\mu$m.

**3.** The use according to claim 1, where the particle size is from 15 to 40 $\mu$m.

**4.** The use according to any of claims 1 to 3, where the hydration capacity is greater than 7.5 g/g.

**5.** The use according to any of claims 1 to 3, where the hydration capacity is greater than 8.0 g/g.

**6.** Tablets comprising a crosslinked polyvinylpyrrolidone according to any of claims 1 to 5 in concentrations of from 0.5 to 50% by weight based on the total weight of the tablet.

**7.** Tablets according to claim 6, comprising a crosslinked polyvinylpyrrolidone in concentrations of from 1.0 to 10% by weight.

**8.** Tablets which rapidly disintegrate in the mouth and comprise a crosslinked polyvinylpyrrolidone according to any of claims 1 to 5 in concentrations of from 1.0 to 10% by weight.

**9.** Tablets which rapidly disintegrate in the mouth according to claim 8, comprising from 20 to 95% by weight of a sugar alcohol.

**Revendications**

**1.** Utilisation de polyvinylpyrrolidone réticulée d'une taille de particule moyenne de 5 à 60 $\mu$m et d'une capacité d'hydratation, déterminée selon le procédé tel que décrit à la page 2, lignes 10 à 16, supérieure à 7 g/g, en tant que délitant de comprimés.

**2.** Utilisation selon la revendication 1, dans laquelle la taille de particule est de 10 à 50 $\mu$m.

**3.** Utilisation selon la revendication 1, dans laquelle la taille de particule est de 15 à 40 $\mu$m.

**4.** Utilisation selon l'une quelconque des revendications 1 à 3, dans laquelle la capacité d'hydratation est supérieure à 7,5 g/g.

**5.** Utilisation selon l'une quelconque des revendications 1 à 3, dans laquelle la capacité d'hydratation est supérieure à 8,0 g/g.

**6.** Comprimés, contenant une polyvinylpyrrolidone réticulée selon l'une quelconque des revendications 1 à 5 en des concentrations de 0,5 à 50 % en poids, par rapport au poids total du comprimé.

**7.** Comprimés selon la revendication 6, contenant une polyvinylpyrrolidone réticulée en des concentrations de 1,0 à 10 % en poids.

8. Comprimés se décomposant rapidement dans la bouche, contenant une polyvinylpyrrolidone réticulée selon l'une quelconque des revendications 1 à 5 en des concentrations de 1,0 à 10 % en poids.

9. Comprimés se décomposant rapidement dans la bouche selon la revendication 8, convenant 20 à 95 % en poids d'un polyol.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005105049 A
- WO 03072084 A
- WO 03032978 A
- EP 1036839 A
- US 6677417 B
- EP 0177812 B

**In der Beschreibung aufgeführte Nicht-Patentliteratur**

- **V. BUEHLER.** Polyvinylpyrrolidone Excipients for Pharmaceuticals. Springer Verlag, 2005, 128 ff
- **V. BUEHLER.** Polyvinylpyrrolidone Excipients for Pharmaceuticals. Springer Verlag, 2005, 132 ff